# FASCICULE DE BREVET EUROPEEN

(11) **EP 4 031 231 B1**
(45) Date de publication et mention de la délivrance du brevet: **11.06.2025**
(21) Numéro de dépôt: 20772312.3
(22) Date de dépôt: 21.09.2020
(51) Int. Cl.: A61M 39/10, A61J 1/14, A61J 1/10

(54) **CONNECTEUR MONOBLOC POUR POCHE DE PERFUSION SOUPLE**
EINSTÜCKIGER VERBINDER FÜR EINEN FLEXIBLEN INFUSIONSBEUTEL
ONE-PIECE CONNECTOR FOR A FLEXIBLE INFUSION BAG

(30) Priorité: 20.09.2019 FR 1910422
(43) Date de publication de la demande: 27.07.2022
(73) Titulaire: Technoflex, 64210 Bidart (FR)
(72) Inventeur: ANGOT, Maxime, 64210 AHETZE (FR); GARCIA, David, 64240 BRISCOUS (FR); PRUVOT, Fabien, 64310 SAINT PEE SUR NIVELLE (FR)
(74) Mandataire: Regimbeau
(86) Numéro de dépôt international: PCT/EP2020/076322
(87) Numéro de publication internationale: WO 2021/053237

(56) Documents cités:
- ES-T3- 2 383 106
- FR-A1- 2 948 289
- JP-B2- 3 241 590
- US-A1- 2005 035 149
- US-A1- 2013 237 946

## Description

### Domaine technique

L'invention appartient au domaine des poches de perfusion souples à usage unique.

Plus particulièrement, l'invention concerne les poches souples à base de matières polyoléfines réalisées par soudure thermique présentant au moins un compartiment contenant une solution médicamenteuse et au moins une interface permettant le remplissage et la vidange pour administration au patient.

Plus particulièrement, l'invention concerne un connecteur pour poche souple.

### Technique antérieure

Actuellement, il existe sur le marché diverses solutions d'interfaces de poches de perfusion souples.

L'interface peut par exemple prendre la forme d'un ou plusieurs tubes extrudés soudés sur la poche. Au moins un des tubes présente un connecteur emmanché afin de permettre l'insertion d'un perfuseur pour l'administration de la solution médicamenteuse au patient.

Un inconvénient de cette solution est que la soudure du ou des tubes sur la poche souple est assez complexe et présente potentiellement des problématiques d'esthétisme ou d'intégrité, en particulier si le film plastique est rigide et/ou épais. Par ailleurs, l'interface comporte au moins deux pièces (au moins un tube, et un connecteur), ce qui est coûteux et pose des problèmes en termes de compatibilité chimique avec le médicament et de performances d'étanchéité.

L'interface peut également prendre la forme d'au moins un connecteur éventuellement monobloc soudé sur la poche grâce à l'insertion d'un outil par l'intérieur de la poche. L'insertion de l'outil permet d'assurer un contre-appui évitant l'affaissement du connecteur pendant la soudure.

Un inconvénient de ce type d'interface est que l'insertion de l'outil par l'intérieur de la poche souple risque d'endommager et/ou de contaminer l'intérieur de la poche.

L'interface peut également prendre la forme d'au moins un connecteur soudé sur la poche par l'extérieur, et composé d'un assemblage de plusieurs pièces, de sorte que les diverses parties du connecteur peuvent être en des matières différentes afin de présenter des propriétés mécaniques et des performances de déformation différentes.

Un inconvénient de ce type d'interface est qu'il est coûteux et pose des problèmes en termes de compatibilité chimique avec le médicament et de performances d'étanchéité.

Le document FR 2 948 289 A1 divulgue un port d'injection pour un récipient à usage médical, comportant un bouchon perçable comprenant un matériau auto-obturateur de sorte à éviter d'éventuelles fuites lors du retrait de l'aiguille d'une seringue.

### Exposé de l'invention

L'invention vise à résoudre les inconvénients de l'art antérieur en proposant un connecteur pour l'insertion d'un perfuseur, ledit connecteur étant destiné à être soudé par l'extérieur à un compartiment de poche souple de perfusion.

L'invention concerne un connecteur pour poche souple de perfusion destinée à contenir un liquide médical, ledit connecteur étant réalisé en une matière thermoplastique, et comprenant une zone de soudure et une zone de connexion, la zone de soudure comportant un premier canal traversant ladite zone de soudure, la zone de connexion comprenant une chambre étanche aux micro-organismes présentant un col, un second canal et une interface étanche séparant ladite chambre étanche et ledit second canal de ladite zone de connexion, le second canal étant le prolongement dans la zone de connexion du premier canal de la zone de soudure, les deux canaux définissant un canal d'écoulement de liquides. Selon l'invention, le connecteur est formé en une seule pièce unique de matière thermoplastique et la zone de soudure comprend une zone rigidifiée.

Dans une forme de réalisation, la zone rigidifiée comporte un ensemble de nervures parallèles entre elles.

Dans une forme de réalisation, l'interface étanche est une membrane souple.

Dans une forme de réalisation, la membrane souple a une épaisseur sensiblement comprise entre 0.1 et 0.6 millimètres.

Dans une forme de réalisation, la zone de soudure présente une zone auxiliaire prolongeant la zone rigidifiée sur une partie amont de ladite zone de soudure, de sorte que la zone rigidifiée s'étend entre la zone de connexion et la zone auxiliaire.

Dans une forme de réalisation, la zone auxiliaire est dépourvue de nervures.

Selon l'invention, le col est situé entre une partie amont de la chambre et une partie aval de la chambre et le col présente un diamètre interne inférieur à un premier diamètre interne de la partie amont de la chambre, et inférieur à un deuxième diamètre interne de la partie aval de la chambre.

Dans une forme de réalisation, le premier canal, le second canal et la chambre étanche ont des diamètres internes sensiblement identiques.

Dans une forme de réalisation, la zone de connexion comprend une partie sécable.

Dans une forme de réalisation, la partie sécable s'étend sensiblement entre un plan de sécabilité d'une gorge située en aval de l'interface étanche et du col et une extrémité avale du connecteur.

Dans une forme de réalisation, une distance entre le col et l'interface étanche est sensiblement comprise entre 5 millimètres et 9 millimètres.

Dans une forme de réalisation, le connecteur est réalisé sur une base polyoléfine. C'est-à-dire que le connecteur est réalisé en une matière thermoplastique comprenant un polyoléfine en proportion majoritaire en poids (par exemple un polyéthylène ou un polypropylène).

L'invention concerne également une poche souple de perfusion comportant des parois délimitant un compartiment et un connecteur tel que défini précédemment, dans laquelle les parois sont soudées sur la zone de soudure du connecteur.

Dans une forme de réalisation, la poche souple de perfusion comporte une pièce injectée intégrant le connecteur tel que défini précédemment et au moins un élément d'interface supplémentaire.

Dans une forme de réalisation, l'au moins un élément d'interface supplémentaire est un tube permettant le remplissage de la poche de perfusion.

### Brève description des dessins

La figure 1 représente un connecteur selon l'invention, en vues de face, à gauche, et de profil, à droite.
La figure 2 représente le connecteur selon l'invention, dans le mode de la réalisation de la figure 1, en vue de coupe dans le plan de coupe A - A.
La figure 3A représente un détail de la zone de soudure du connecteur selon l'invention, dans un premier mode de réalisation, ainsi que d'une partie de la zone de connexion, dans le mode de la réalisation de la figure 1.
La figure 3B représente un détail de la zone de soudure du connecteur selon l'invention, ainsi que d'une partie de la zone de connexion, dans le mode de la réalisation de la figure 3A, en vue de coupe dans le plan de coupe B - B.
La figure 3C représente un détail de la zone de soudure du connecteur selon l'invention, ainsi que d'une partie de la zone de connexion, dans le mode de la réalisation de la figure 3A, en vue de coupe dans le plan de coupe C - C.
La figure 4 représente un détail de la zone de connexion du connecteur selon l'invention, dans un mode de réalisation dans lequel la gorge a un profil en biseau, avant soudure thermique de l'extrémité avale.
La figure 5 représente l'étape de soudure thermique du connecteur selon l'invention au compartiment de la poche souple et une position relative recherchée entre le connecteur, les films du compartiment et les outils de soudure pour cette étape de soudure thermique.
La figure 6A représente une position relative entre le connecteur, les films du compartiment et les outils de soudure, lors de l'étape de soudure thermique du connecteur selon l'invention au compartiment de la poche souple, dans une configuration dans laquelle les outils de soudure sont décentrés en amont du connecteur par rapport à la position de la figure 5.
La figure 6B représente une position relative entre le connecteur, les films du compartiment et les outils de soudure, lors de l'étape de soudure thermique du connecteur selon l'invention au compartiment de la poche souple, dans une configuration dans laquelle les outils de soudure sont décentrés en aval du connecteur par rapport à la position de la figure 5.
La figure 7 représente une poche souple comportant un compartiment et une pièce injectée intégrant un connecteur selon l'invention et un tube permettant de remplir le compartiment.
La figure 8A représente une première vue en perspective d'un second mode de réalisation de la zone de soudure du connecteur.
La figure 8B représente une seconde vue en perspective de la zone de soudure du connecteur dans le mode de réalisation de la figure 8A.
La figure 8C représente une vue de face de la zone de soudure du connecteur dans le mode de réalisation de la figure 8A, ainsi qu'une partie de la zone de connexion.
La figure 8D représente une vue de section, dans le plan D - D de la zone de soudure du connecteur dans le mode de réalisation de la figure 8A.
La figure 8E représente une vue de section, dans le plan E - E de la zone de soudure du connecteur dans le mode de réalisation de la figure 8A.
La figure 8F représente une vue de section, dans le plan F - F de la zone de soudure du connecteur dans le mode de réalisation de la figure 8A.

### Description détaillée

L'invention sera mieux comprise à la lecture de la description qui suit et à l'examen des figures qui l'accompagnent. Celles-ci ne sont présentées qu'à titre indicatif et nullement limitatif de l'invention.

Une poche souple de perfusion selon un mode de réalisation de l'invention comporte des parois 3 délimitant un compartiment 5 et un connecteur 1.

Les parois 3 du compartiment 5 et le connecteur 1 sont réalisées en matière thermoplastique médicalement inerte. Avantageusement, le connecteur 1 est réalisé en polyoléfine (tel que par exemple en polyéthylène ou en polypropylène), qui présente une très bonne inertie chimique. Dans tous les cas, le matériau plastique est compatible avec les procédés de transformation de matières thermoplastiques.

Chaque paroi 3 comprend un film en matière plastique. Le film contient de préférence au moins une polyoléfine en proportion majoritaire en poids. Le film peut être un film multicouches. Les parois 3 du compartiment 5 sont assemblées entre elles par soudure thermique. Le connecteur 1 est monobloc, réalisé par injection thermoplastique et soudé aux parois du compartiment par l'extérieur, c'est-à-dire qu'aucun outil n'est placé à l'intérieur du compartiment pour réaliser la soudure du connecteur 1 aux parois.

On entend par « monobloc » que le connecteur est d'un seul tenant, c'est-à-dire qu'il est formé en une seule pièce unique de matériau par moulage par injection dans un même moule, et qu'il ne résulte pas d'un assemblage de pièces distinctes.

Le connecteur 1 peut également être réalisé par moulage par bi-injection, c'est-à-dire par injection de plusieurs matériaux thermoplastiques dans un même moule.

En référence aux figures, le connecteur 1 présente une zone de soudure 10 et une zone de connexion 11. La zone de soudure 10 comporte une zone rigidifiée 101 au niveau de laquelle est sensiblement réalisée la soudure thermique entre les parois du compartiment 5 et le connecteur 1. Ainsi, les parois du compartiment 5 sont soudées sur la zone rigidifiée 101 du connecteur 1. Une rigidité de la zone rigidifiée 101 est supérieure à une rigidité de la zone de connexion 11. La rigidité de la zone rigidifiée 101 est telle qu'elle permet de réaliser la soudure thermique des parois 3 sans que ladite zone de soudure 10 ne s'affaisse sous l'effet des efforts exercés par les outils de soudure 2. Par exemple, la zone rigidifiée 101 est capable d'opposer une force supérieure ou égale à 90 Newtons lorsqu'elle subit un écrasement de 2 millimètres. La présence de cette zone rigidifiée 101 permet ainsi d'éviter l'insertion dans le compartiment 5 d'un outil supplémentaire pour éviter un tel affaissement par reprise des efforts, un tel outil risquant d'endommager ou de contaminer la poche.

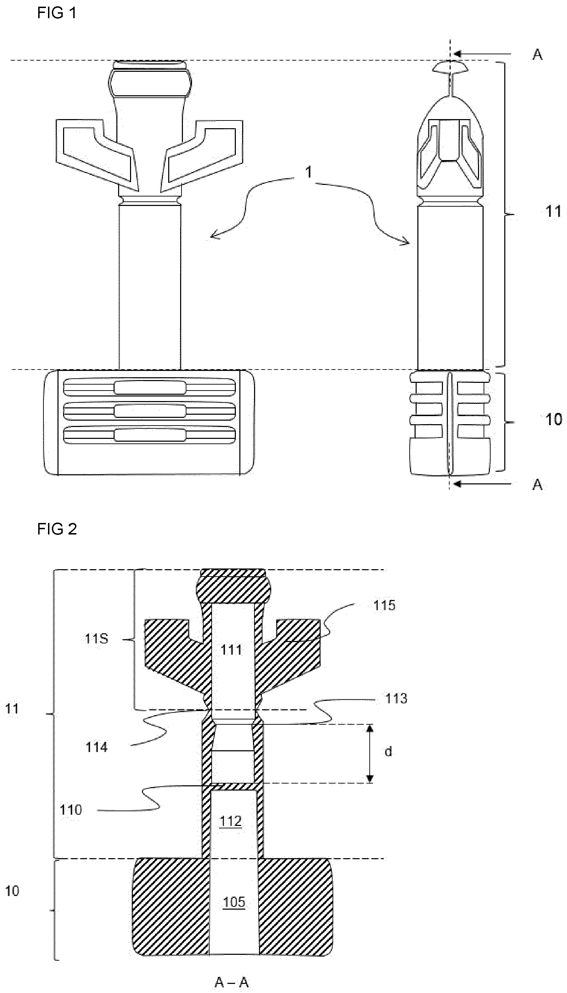

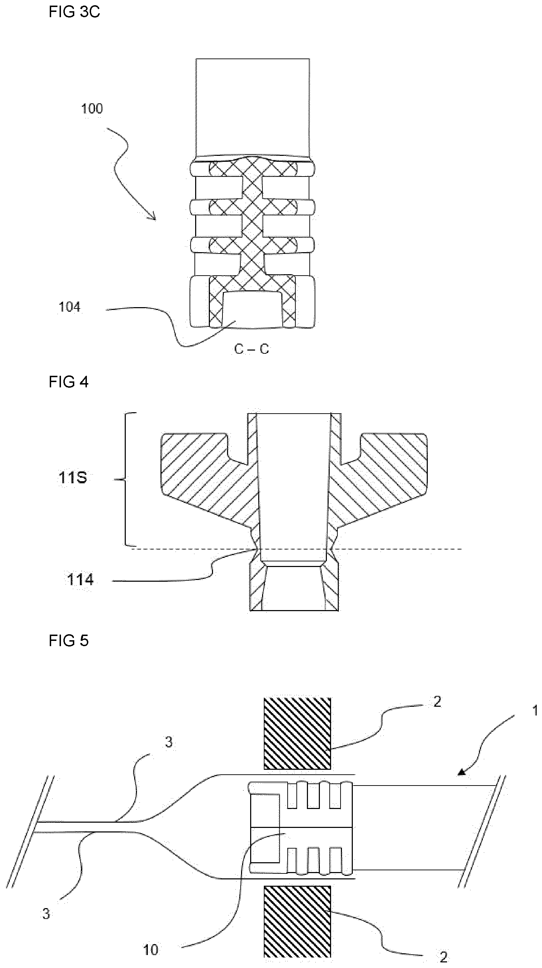

Dans une première forme de réalisation illustrée sur la figure 1, la zone rigidifiée 101 présente sur des bords latéraux des ailettes radiales 100, et sur une partie centrale une zone nervurée conférant à la zone rigidifiée 101 une rigidité accrue. La zone nervurée comporte un ensemble de nervures 102 parallèles entre elles et s'étendant d'une ailette 100 à une autre. Les nervures 102, disposées sur des surfaces extérieures de la zone de soudure 10, sont orientées dans un axe des efforts exercés par les outils de soudure 2 représentés sur la figure 5, de sorte à faciliter la soudure du connecteur 1 aux parois du compartiment, les efforts exercés par les outils de soudure 2 étant repris par les nervures, sans nécessiter l'insertion d'un outil supplémentaire dans le compartiment, comme mentionné plus haut. Une largeur L des nervures est de l'ordre du millimètre, par exemple environ égale à 1.2 millimètres.

Un premier canal 105 traverse la zone de soudure 10 selon une direction perpendiculaire à celle des nervures 102. Le premier canal 105 présente une forme cylindrique de révolution ayant un premier axe. Le premier canal 105 est débouchant au niveau d'une extrémité amont de la zone de soudure, le terme « amont » se référant au sens d'écoulement du liquide lorsque la poche souple de perfusion est utilisée pour administrer la solution médicamenteuse contenue dans le compartiment 5 à un patient.

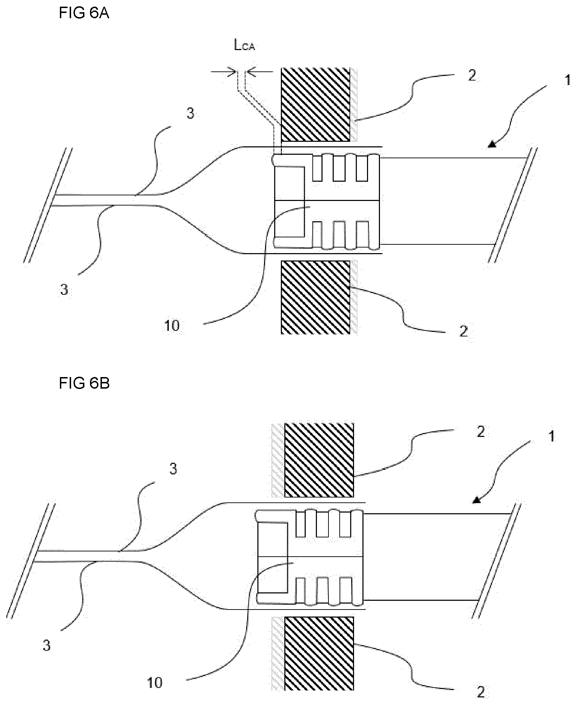

Dans un mode de réalisation avantageux de l'invention, la zone de soudure 10 présente sur une partie amont une zone auxiliaire 103 prolongeant la zone rigidifiée 101. En référence à la figure 5 représentant la position relative recherchée pour la soudure, entre le connecteur 1, les outils de soudure 2 et les parois 3, la présence d'une zone auxiliaire 103 permet notamment d'assurer la présence d'une surface de contre-appui non soudée pour que le film formant la paroi 3 du compartiment reste orienté en amont dans le sens de la soudure, si, comme illustré sur la figure 6A, les outils de soudure se retrouvent décentrés plus en amont du connecteur 1 par rapport à la figure 5.

Sur les figures 6A et 6B, les outils de soudure en gris clair illustrent la position des outils de soudure 2 tels que positionnés dans la configuration de la figure 5.

La zone auxiliaire 103 présente une partie évidée 104 au niveau d'une partie amont de la zone de soudure afin que l'épaisseur de parois de ladite zone auxiliaire soit relativement faible, par exemple inférieure à 3 millimètres afin d'avoir sur l'ensemble du connecteur des épaisseurs suffisamment homogènes pour limiter voire éviter les défauts lors de la fabrication par injection thermoplastique du connecteur 1.

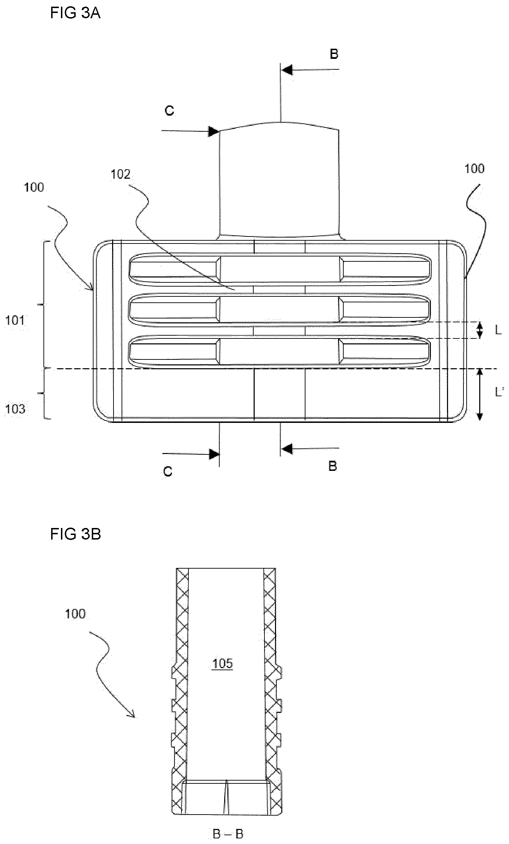

Dans le mode de réalisation de la figure 1, et en référence également à la figure 3A, la zone auxiliaire 103 s'étend sur une largeur L' sensiblement au moins égale à la somme de :
- la largeur L d'une nervure de la zone nervurée,
- l'intervalle de tolérance de positionnement des outils de soudure (typiquement comprise entre 0 et 4 millimètres), et
- une largeur minimale L_{CA} de surface de contre-appui tel qu'illustré sur la figure 6A (cette largeur minimale L_{CA} est comprise entre 0,5 et 2 millimètres).

Typiquement, la largeur L' de la zone auxiliaire 103, mesurée parallèlement au premier axe, est supérieure ou égale à 2,5 millimètres. La zone auxiliaire 103 est dépourvue de nervures.

La surface de contre-appui correspond à une zone de la zone auxiliaire 103 sur laquelle les parois du compartiment sont amenées à reposer sans être soudées, dans le prolongement de la partie des parois soudées au connecteur, ce qui permet d'éviter des contraintes sur les films formant les parois pouvant entraîner leur détérioration du fait de leur fragilité. Dans l'exemple illustré, dans lequel les nervures 102 ont une largeur de 1,2 millimètres et pour laquelle il est considéré une tolérance de positionnement des outils de soudure de ±1 millimètre (c'est-à-dire un intervalle de tolérance égal à 2 millimètres), la zone non nervurée 103 s'étend sur une largeur L' de 4,2 millimètres environ, incluant une surface de contre-appui de largeur L_{CA} égale à 1 millimètre.

En plus d'assurer la présence d'une surface de contre-appui comme évoqué plus haut, une telle largeur L' permet d'assurer une surface soudée au moins égale à celle d'une nervure de la zone rigidifiée 101 si, comme illustré sur la figure 6B, les outils de soudure se retrouvent décentrés plus en aval du connecteur par rapport à la figure 5.

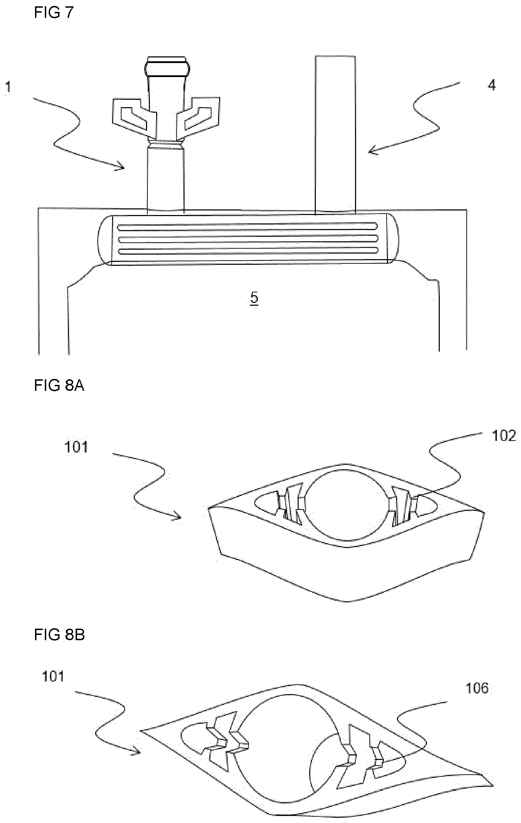

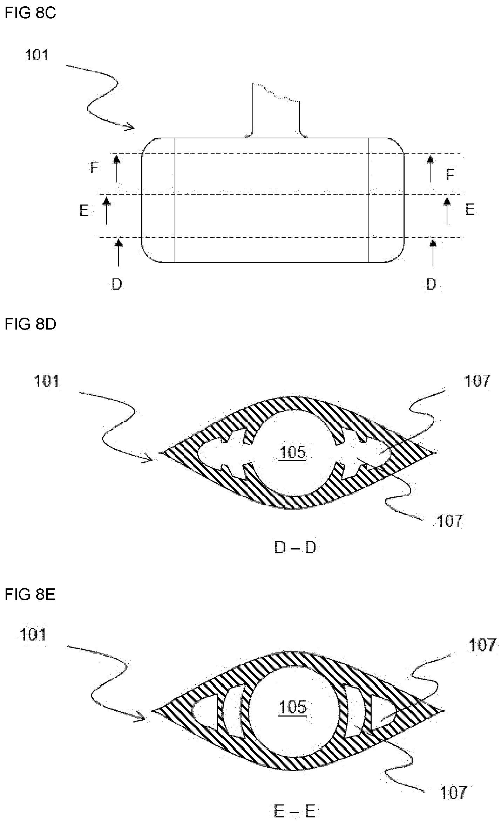

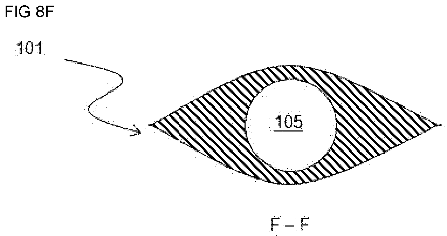

Les figures 8A-8D, représentant des vues en perspective et de section de la zone de soudure 10 d'un connecteur, illustrent un second mode de réalisation de la zone de soudure. Ici, la zone auxiliaire 103 n'est pas représentée, la zone de soudure 10 correspond donc à la zone rigidifiée 101. Toutefois, de même que décrit précédemment dans le premier mode de réalisation, une zone auxiliaire 103 pourrait être ajoutée en amont de la zone rigidifiée 101 représentée.

Dans ce mode de réalisation, la zone rigidifiée 101 présente des nervures disposées sur des surfaces intérieures de la zone de soudure 10 et s'étendant dans une direction d'écoulement du liquide, lorsque la poche souple est en service. Les nervures s'étendent d'une surface intérieure à la surface intérieure opposée.

Comme illustré sur les figures 8A, 8B et 8D, à proximité de l'extrémité amont de la zone rigidifiée 101, les nervures présentent des encoches 106 afin d'établir une communication entre le premier canal 105 et les compartiments latéraux 107 formés par les nervures, pour permettre une bonne circulation du liquide.

L'homme du métier comprendra que la rigidité de la zone rigidifiée 101 pourra être assurée de diverses manières et que les modes de réalisation présentés ne sont en aucun cas limitatif de l'invention. Par exemple, l'épaisseur des parois de la zone rigidifiée pourra être adaptée pour fournir à la zone rigidifiée la rigidité souhaitée, en tenant compte toutefois des contraintes liées à la réalisation du connecteur par injection thermoplastique. Le choix du matériau utilisé pour réaliser le connecteur pourra également influencer cette rigidité.

Il convient de noter également que la rigidité de la zone auxiliaire 103 n'est pas nécessairement la même que celle de la zone rigidifiée 101, dans la mesure où la zone auxiliaire 103 n'est pas amenée à reprendre les efforts des outils de soudure, ou alors dans une moindre mesure. La rigidité de la zone auxiliaire 103 pourra par exemple être inférieure à celle de la zone rigidifiée 101.

La zone de connexion 11 présente une interface étanche 110 séparant une chambre 111 étanche aux micro-organismes et un second canal 112. Le second canal 112 présente une forme cylindrique de révolution ayant un second axe. Le second canal 112 est le prolongement dans la zone de connexion 11 du premier canal 105 de la zone de soudure 10. Le second axe du second canal 112 est colinéaire avec le premier axe du premier canal 105. De plus, le premier canal 105 débouche dans le second canal 112. Les deux canaux 105, 112 ont, dans les modes de réalisation illustrés, des diamètres sensiblement identiques. Les diamètres des canaux 105 et 112 sont chacun compris entre 5 et 8 millimètres. Les canaux 105, 112 définissent ainsi un canal d'écoulement des liquides depuis l'intérieur du compartiment de la poche souple de perfusion vers l'extérieur du compartiment.

L'interface étanche 110 destinée à être transpercée par un perfuseur (non représenté) est une membrane souple s'étendant transversalement à l'axe du second canal 112. La membrane souple présente une épaisseur sensiblement comprise entre 0.1 et 0.6 millimètres. Une épaisseur de membrane souple inférieure à 0.1 millimètre rendrait la membrane trop fragile d'une part, et d'autre part ne permettrait pas sa fabrication par injection. Une épaisseur supérieure à 0.6 millimètre nécessiterait une force trop importante pour insérer le perfuseur dans le connecteur.

La chambre étanche 111 est une zone évidée de la zone de connexion, fermée par soudure thermique au niveau d'une extrémité avale de ladite chambre, correspondant à une extrémité avale du connecteur, après réalisation du connecteur monobloc par injection thermoplastique, et fermée à son extrémité amont par la membrane souple 110. La chambre étanche présente une forme cylindrique de révolution ayant un troisième axe. De plus, la chambre étanche 111 présente un col 113 ayant un diamètre inférieur au diamètre du reste de la chambre étanche de sorte à assurer une étanchéité par mise en compression radiale dudit col lors de l'insertion du perfuseur dans la chambre étanche. Le col 113 forme un étranglement. Autrement dit, le col 113 présente un diamètre à la fois inférieur à un premier diamètre interne d'une partie amont de la chambre s'étendant en amont du col 113, et inférieur à un deuxième diamètre interne d'une partie aval de la chambre s'étendant en aval du col 113. Le diamètre du col 113 est compris entre 4 et 6 millimètres, par exemple égal à 5 millimètres environ. Le premier diamètre interne de la partie amont de la chambre et le deuxième diamètre interne de la partie aval de la chambre peuvent être compris chacun entre 5 et 8 millimètres, par exemple égal à 6,5 millimètres environ.

Les diamètres internes de la chambre étanche 111 et du second canal 112 sont tels qu'ils permettent un jeu entre le connecteur 1 et le perfuseur (en-dehors de la zone du col 113) lorsque celui-ci est inséré dans ledit connecteur. Un avantage d'un tel jeu est de permettre de maîtriser la force nécessaire à l'insertion du perfuseur, la résistance à l'insertion du perfuseur provenant uniquement de la compression radiale du col et de la perforation axiale de la membrane.

La zone de connexion 11 présente une partie sécable 11S s'étendant sensiblement entre un plan de sécabilité d'une gorge 114 située en aval de l'interface étanche 110 et du col 113, et une extrémité avale du connecteur 1, le terme « aval(e) » se référant au sens d'écoulement du liquide lorsque la poche souple de perfusion est utilisée pour administrer la solution médicamenteuse au patient. On entend par « plan de sécabilité » un plan dans lequel la zone de connexion 11 est fragilisée pour permettre une séparation manuelle de ladite partie sécable du reste de la zone de connexion 11 par un opérateur. En pratique cette fragilisation est apportée par une épaisseur de la paroi de la zone de connexion 11 minimale au niveau de la gorge 114. En référence à la figure 4, la gorge 114 présente une forme annulaire et s'étend sur une surface externe de la paroi de la zone de connexion. La gorge 114 peut présenter un profil en biseau.

De manière connue, la partie sécable 11S est détachée du reste du connecteur lors de la mise en œuvre de la poche de perfusion, afin d'ouvrir la chambre étanche 111 et de permettre l'insertion du perfuseur qui vient percer l'interface étanche 110.

Une distance d entre un plan du col 113 et un plan de l'interface étanche 110 est préférentiellement comprise entre 5 et 9 millimètres. En effet, en-deçà de 5 millimètres, il y a un risque important que le col n'assure pas l'étanchéité avec le perfuseur, par compression radiale, lorsque l'interface étanche 110 est percée par la pointe du perfuseur, en particulier avec des perfuseurs présentant une pointe biseautée très longue. Au-delà de 9 millimètres, il y a un risque important que la garde d'enfoncement du perfuseur atteigne la surface d'appui du connecteur, ladite surface d'appui correspondant à la surface formée sur le connecteur au niveau de la gorge 114 après retrait de la partie sécable 11S, avant que l'interface étanche n'ait été complètement percée par la pointe du perfuseur, en particulier si le perfuseur présente une faible distance pointe-garde. Bien entendu, il convient de noter que ces valeurs limites de distance, valables pour une majorité de perfuseurs, peuvent être adaptées au cas par cas suivant le perfuseur utilisé.

La partie sécable 11S présente par ailleurs des éléments de préhension radiaux 115 permettant de saisir la partie sécable pour la séparer du reste de la zone de connexion 11 par rotation de ladite partie sécable par rapport au reste de la zone de connexion. Le perfuseur peut ensuite être inséré pour percer l'interface étanche 110 et être inséré dans le canal d'écoulement.

Le connecteur 1 selon l'invention est soudé thermiquement aux parois 3 composant le compartiment de la poche souple selon le schéma de la figure 5. Les outils 2 viennent se placer autour des parois 3 et de la zone de soudure 10 afin de souder les parois 3 et le connecteur 1 entre eux par conduction thermique. Les nervures 102 permettent à la zone de soudure 10 de ne pas s'affaisser lors de la réalisation de la soudure thermique, du fait des efforts exercés par les outils sur le connecteur.

Le connecteur selon l'invention présente les avantages suivants :
- il est soudé sur les parois du compartiment de la poche souple sans insérer d'outil à l'intérieur dudit compartiment,
- il peut être interfacé avec des perfuseurs conformément à la norme ISO 15747 (pour les perfuseurs conformes à l'ISO 8536 et une majorité des perfuseurs américains non normés),
- il permet de fermer la chambre étanche par une soudure pour la rendre imperméable aux micro-organismes, et ainsi assurer une interface stérile à travers laquelle insérer le perfuseur après retrait de la partie sécable,
- il est inerte vis-à-vis des solutions placées dans la poche souple.

En particulier, le fait que le connecteur soit monobloc permet de :
- limiter les risques d'erreurs d'assemblage,
- optimiser les coûts de fabrication,
- limiter les risques d'incompatibilité chimique et biologique entre la solution liquide et la poche souple.

En référence à la figure 7, le connecteur 1 selon un mode de réalisation de l'invention peut être intégré à une pièce injectée de plus grande dimension comprenant des éléments d'interface supplémentaires en plus du connecteur, par exemple un tube 4 permettant le remplissage de la poche par le laboratoire. Dans le mode de réalisation illustré sur la figure 7, la zone de soudure 10 du connecteur est plus large pour pouvoir accueillir le tube, tous ces éléments formant une pièce injectée monobloc.

## Revendications

1. Connecteur (1) pour poche souple de perfusion destinée à contenir un liquide médical, ledit connecteur étant réalisé en au moins une matière thermoplastique, et comprenant une zone de soudure (10) et une zone de connexion (11), la zone de soudure (10) comportant un premier canal (105) traversant ladite zone de soudure, la zone de connexion (11) comprenant une chambre (111) étanche aux micro-organismes présentant un col (113), un second canal (112) et une interface étanche (110) séparant ladite chambre étanche et ledit second canal de ladite zone de connexion, le second canal (112) étant le prolongement dans la zone de connexion (11) du premier canal (105) de la zone de soudure (10), les deux canaux (105, 112) définissant un canal d'écoulement de liquides, ledit connecteur étant formé en une seule pièce unique de matière thermoplastique et la zone de soudure (10) comprenant une zone rigidifiée (101), le col (113) étant situé entre une partie amont de la chambre et une partie aval de la chambre et le col (113) présentant un diamètre interne inférieur à un premier diamètre interne de la partie amont de la chambre, et inférieur à un deuxième diamètre interne de la partie aval de la chambre (111) .

2. Connecteur selon la revendication 1, dans lequel la zone rigidifiée comporte un ensemble de nervures (102) parallèles entre elles.

3. Connecteur selon la revendication 1 ou la revendication 2, dans lequel la zone de soudure (10) présente une zone auxiliaire (103) prolongeant la zone rigidifiée (101) sur une partie amont de ladite zone de soudure, de sorte que la zone rigidifiée (101) s'étend entre la zone de connexion (11) et la zone auxiliaire (103).

4. Connecteur selon la revendication 3, dans lequel la zone auxiliaire (103) est dépourvue de nervures.

5. Connecteur selon l'une quelconque des revendications précédentes, dans lequel l'interface étanche (110) est une membrane souple d'épaisseur sensiblement comprise entre 0.1 et 0.6 millimètres.

6. Connecteur selon l'une quelconque des revendications précédentes, dans lequel la zone de connexion (11) comprend une partie sécable (11S) s'étendant sensiblement entre un plan de sécabilité d'une gorge (114) située en aval de l'interface étanche (110) et du col (113) et une extrémité avale du connecteur (1).

7. Connecteur selon l'une quelconque des revendications précédentes, dans lequel une distance entre le col (113) et l'interface étanche (110) est sensiblement comprise entre 5 millimètres et 9 millimètres.

8. Connecteur selon l'une quelconque des revendications précédentes, le connecteur étant réalisé sur une base polyoléfine.

9. Poche souple de perfusion comportant des parois (3) délimitant un compartiment (5) et un connecteur (1) selon l'une quelconque des revendications précédentes, dans laquelle les parois (3) sont soudées sur la zone de soudure (10) du connecteur (1).

10. Poche souple de perfusion selon la revendication 9, comportant en outre une pièce injectée intégrant le connecteur (1) et au moins un élément d'interface supplémentaire.

11. Poche souple de perfusion selon la revendication 10, dans laquelle l'au moins un élément d'interface supplémentaire est un tube permettant le remplissage de la poche de perfusion.

## Patentansprüche

1. Verbinder (1) für einen flexiblen Infusionsbeutel, der zur Aufnahme einer medizinischen Flüssigkeit bestimmt ist, wobei der Verbinder aus mindestens einem thermoplastischen Material hergestellt ist und einen Schweißbereich (10) und einen Verbindungsbereich (11) umfasst, wobei der Schweißbereich (10) einen ersten Kanal (105) aufweist, der den Schweißbereich durchquert, wobei der Verbindungsbereich (11) eine gegen Mikroorganismen dichte Kammer (111) umfasst, die einen Hals (113), einen zweiten Kanal (112) und eine dichte Schnittstelle (110) aufweist, die die dichte Kammer und den zweiten Kanal vom Verbindungsbereich trennt, wobei der zweite Kanal (112) die Verlängerung des ersten Kanals (105) des Schweißbereichs (10) in den Verbindungsbereich (11) ist, wobei die beiden Kanäle (105, 112) einen Flüssigkeitsströmungskanal bilden, wobei der Verbinder aus einem einzigen Stück thermoplastischen Materials gebildet ist und der Schweißbereich (10) einen versteiften Bereich (101) umfasst, wobei sich der Hals (113) zwischen einem stromaufwärts gelegenen Teil der Kammer und einem stromabwärts gelegenen Teil der Kammer befindet und der Hals (113) einen Innendurchmesser aufweist, der kleiner ist als ein erster Innendurchmesser des stromaufwärts gelegenen Teils der Kammer und kleiner als ein zweiter Innendurchmesser des stromabwärts gelegenen Teils der Kammer (111).

2. Verbinder nach Anspruch 1, wobei der versteifte Bereich eine Anordnung von zueinander parallelen Rippen (102) aufweist.

3. Verbinder nach Anspruch 1 oder Anspruch 2, wobei der Schweißbereich (10) einen Hilfsbereich (103) aufweist, der den versteiften Bereich (101) an einem stromaufwärts gelegenen Teil des Schweißbereichs verlängert, so dass sich der versteifte Bereich (101) zwischen dem Verbindungsbereich (11) und dem Hilfsbereich (103) erstreckt.

4. Verbinder nach Anspruch 3, wobei der Hilfsbereich (103) keine Rippen aufweist.

5. Verbinder nach einem der vorstehenden Ansprüche, wobei die dichte Schnittstelle (110) eine flexible Membran mit einer Dicke ist, die im Wesentlichen zwischen 0,1 und 0,6 Millimetern liegt.

6. Verbinder nach einem der vorstehenden Ansprüche, wobei der Verbindungsbereich (11) einen trennbaren Teil (11S) umfasst, der sich im Wesentlichen zwischen einer Trennebene einer Nut (114) erstreckt, die sich stromabwärts von der dichten Schnittstelle (110) und dem Hals (113) und einem stromabwärts gelegenen Ende des Verbinders (1) befindet.

7. Verbinder nach einem der vorstehenden Ansprüche, wobei ein Abstand zwischen dem Hals (113) und der dichten Schnittstelle (110) im Wesentlichen zwischen 5 Millimetern und 9 Millimetern liegt.

8. Verbinder nach einem der vorstehenden Ansprüche, wobei der Verbinder auf einer Polyolefinbasis hergestellt ist.

9. Flexibler Infusionsbeutel, der Wände (3) aufweist, die eine Kammer (5) begrenzen, und einen Verbinder (1) nach einem der vorstehenden Ansprüche, wobei die Wände (3) am Schweißbereich (10) des Verbinders (1) verschweißt sind.

10. Flexibler Infusionsbeutel nach Anspruch 9, der ferner ein Spritzgussteil aufweist, das den Verbinder (1) und mindestens ein zusätzliches Schnittstellenelement integriert.

11. Flexibler Infusionsbeutel nach Anspruch 10, wobei das mindestens eine zusätzliche Schnittstellenelement ein Schlauch ist, der das Befüllen des Infusionsbeutels gestattet.

## Claims

1. Connector (1) for a flexible infusion bag intended to contain a medical liquid, the connector being made of at least one thermoplastic material and comprising a weld zone (10) and a connection zone (11), the weld zone (10) comprising a first channel (105) passing through the weld zone, the connection zone (11) comprising a chamber (111) impermeable to micro-organisms and having a neck (113), a second channel (112) and a sealed interface (110) separating the impermeable chamber and the second channel from the connection zone, the second channel (112) being the extension into the connection zone (11) of the first channel (105) of the weld zone (10), the two channels (105, 112) defining a liquid flow channel, the connector being formed in a single piece of thermoplastic material and the weld zone (10) comprising a stiffened zone (101), the neck (113) being located between an upstream part of the chamber and a downstream part of the chamber and the neck (113) having an internal diameter smaller than a first internal diameter of the upstream part of the chamber, and smaller than a second internal diameter of the downstream part of the chamber (111) .

2. A connector as claimed in claim 1, in which the stiffened zone comprises a set of ribs (102) parallel to one another.

3. A connector according to claim 1 or claim 2, in which the weld zone (10) has an auxiliary zone (103) extending the stiffened zone (101) over an upstream part of the weld zone, so that the stiffened zone (101) extends between the connection zone (11) and the auxiliary zone (103).

4. A connector as claimed in claim 3, in which the auxiliary zone (103) has no ribs.

5. A connector according to any one of the preceding claims, in which the sealed interface (110) is a flexible membrane with a thickness substantially between 0.1 and 0.6 millimetres.

6. A connector according to any one of the preceding claims, wherein the connection zone (11) comprises a breakable portion (11S) extending substantially between a breakable plane of a groove (114) located downstream of the sealed interface (110) and the neck (113) and a downstream end of the connector (1).

7. A connector according to any one of the preceding claims, wherein a distance between the neck (113) and the sealed interface (110) is substantially between 5 millimetres and 9 millimetres.

8. Connector according to any one of the preceding claims, the connector being made on a polyolefin base.

9. Flexible infusion bag comprising walls (3) delimiting a compartment (5) and a connector (1) according to any one of the preceding claims, in which the walls (3) are welded to the weld zone (10) of the connector (1).

10. A flexible infusion bag as claimed in claim 9, further comprising an injection-moulded part integrating the connector (1) and at least one additional interface element.

11. The flexible infusion bag of claim 10, wherein the at least one additional interface element is a tube for filling the infusion bag.
